# EUROPÄISCHE PATENTSCHRIFT

(11) **EP 1 513 790 B1**
(45) Veröffentlichungstag und Bekanntmachung des Hinweises auf die Patenterteilung: **24.12.2008**
(21) Anmeldenummer: 03755940.8
(22) Anmeldetag: 24.05.2003
(51) Int. Cl.: C07C 35/12, C07C 29/76, C07C 29/00

(54) **KOMPAKTIERTES MENTHOL**
COMPACTED MENTHOL
MENTHOL COMPACTE

(30) Priorität: 31.05.2002 DE 10224087
(43) Veröffentlichungstag der Anmeldung: 16.03.2005
(73) Patentinhaber: Symrise GmbH & Co. KG, 37603 Holzminden (DE)
(72) Erfinder: KÖRBER, Alfred, 37603 Holzminden (DE)
(74) Vertreter: Eisenführ, Speiser & Partner
(86) Internationale Anmeldenummer: PCT/EP2003/005452
(87) Internationale Veröffentlichungsnummer: WO 2003/101924

(56) Entgegenhaltungen:
- DE-A- 2 109 456
- DE-A- 2 530 481
- US-A- 2 760 993
- US-A- 3 023 253
- FRED E. WRIGHT: "The crystallization of menthol." JOURNAL OF THE AMERICAN CHEMICAL SOCIETY., Bd. 39, Nr. 8, 1917, Seiten 1515-1524, XP002256872 DC US in der Anmeldung erwähnt

## Beschreibung

Die Erfindung betrifft Menthol-Presslinge, ein Verfahren zur Herstellung von Menthol- Presslingen und deren Verwendung.

L-Menthol hat einen einzigartigen erfrischenden Geschmack, einen minzigen Geruch und eine stark wirkende Kühlung auf Haut und Schleimhaut. Es wird beispielsweise bei der Mundpflege, in kosmetischen und pharmazeutischen Präparaten, im Tabak und in Süßwaren verwendet, wie z.B. in Perfumer&Flavorist, Vol. 13, October-November 1988, S. 37 beschrieben.

L-Menthol ist der Hauptbestandteil der Pfefferminzöle aus Mentha arvensis (Gehalt: 70 bis 80 %) und Mentha piperita (Gehalt: 50 bis 60 %). Aus dem rohen Pfefferminzöl wird 1-Menthol durch Kristallisation gewonnen. Je nach Kristallisationsverfahren und Ausgangsmaterial unterscheiden sich die Kristalle in Geschmack sowie Kristallgröße und -form. (Perfumer&Flavorist; Vol. 22, November-December 1997, S. 1).

Es sind viele Verfahren zur Herstellung von synthetischem Menthol bekannt. Eine wirtschaftliche Herstellung von synthetischem 1-Menthol geht beispielsweise von Thymol aus. Aus den durch Hydrierung gebildeten vier stereoisomeren Mentholen wird 1-Menthol über mehrere Verfahrensschritte in einer chemischen Reinheit von >99 % und einer Enantiomerenreinheit von >99 % erhalten (z.B. in Bauer, Garbe, Surburg, Common Fragrance and Flavor Materials, 4th Ed., Wiley-VCH, Weinheim 2001, S. 52-55). Das aus diesem Verfahren resultierende 1-Menthol zeichnet sich sensorisch durch seine Reinheit und Strahlkraft aus.

L-Menthol und racemisches Menthol sind in diversen festen Formen im Markt erhältlich, üblich sind beispielsweise Pulver, Kristalle, erstarrtes Destillat, Schuppen und Presslinge. Bisher bekannte Menthol-Presslinge verklumpen beim Lagern nach einiger Zeit, wodurch diese ihre Schüttfähigkeit und Handhabbarkeit einbüßen.

In US-A 3064311 wird 1-Menthol in geschuppter Form beschrieben. Dazu wird destilliertes 1-Menthol geschmolzen und eine dünne geschmolzene Filmschicht auf eine unterkühlte Oberfläche gebracht. Der erstarrte 1-Menthol Film wird in kleine Teile gebrochen. Das Produkt dieses Verfahrens ist eine brüchige 1-Menthol Schuppe, die eine Dicke von 0,125 bis 1,25 mm und eine Ausdehnung von 3 bis 25 mm aufweist. Das so hergestellte geschuppte 1-Menthol zeigt, wie in der Schrift dargelegt, nach 24 Stunden Anhaften und Verklumpen der Schuppen.

Bekannt sind Presslinge aus 1-Menthol, die jedoch, je nach Lagerzeit und Lagerbedingungen, nach einiger Zeit ein Verbacken oder Verklumpen zeigen können. Diese 1-Menthol Presslinge werden aus geschupptem 1-Menthol oder aus Mischungen von geschupptem 1-Menthol und kristallisiertem 1-Menthol hergestellt, wobei der Gesamtgehalt an alpha-1-Menthol unterhalb von 50 Gew.-% liegt.

Aufgabe der vorliegenden Erfindung war daher die Bereitstellung von Menthol-Presslingen, welche eine hohe Lagerstabilität, eine einfache Handhabbarkeit sowie eine gute Schüttbarkeit gewährleisten.

Gegenstand der vorliegenden Erfindung sind daher Menthol-Presslinge, dadurch gekennzeichnet, dass der Gehalt an alpha-Menthol im Pressling bei größer oder gleich 70 Gew.-% liegt.

Ein weiterer Gegenstand der vorliegenden Erfindung ist ein Verfahren zur Herstellung von Menthol-Presslingen, dadurch gekennzeichnet, dass das zur Herstellung der Presslinge eingesetzte Menthol eine Gehalt an alpha-Menthol von mindestens 70 Gew.-% aufweist.

Ein weiterer Gegenstand der vorliegenden Erfindung ist die Verwendung dieser Presslinge zur Einarbeitung in Produkte, speziell zur Aromatisierung von Produkten, insbesondere von Lebensmitteln, Rauchwaren, Riechstoffmischungen, Mundhygieneprodukten, sowie kosmetischen, pharmazeutischen und dermatologischen Produkten.

Alpha-Menthol ist an sich bekannt und ausführlich beispielsweise in J. Am. Chem. Soc. 1917, S. 1515 oder auch in J. Chem. Soc. 101, S. 118 beschrieben und weist einen Schmelzpunkt von 42 bis 43°C auf.

Die Herstellung von alpha-Menthol ist ebenfalls bekannt (z.B. aus DE-A 2109456 oder DE-A 2530481). Darüber hinaus ist nach diesem Verfahren kristallisiertes 1-Menthol in Form weißer Kristalle kommerziell verfügbar (Haarmann & Reimer, Holzminden).

Bevorzugt ist ein Gehalt an alpha-Menthol im Pressling von größer oder gleich 80 Gew.-%, besonders bevorzugt von größer oder gleich 90 Gew.-% und ganz besonders bevorzugt von größer oder gleich 95 Gew.-%. In einer bevorzugten Ausführungsform liegt der Gehalt an alpha-Menthol im Pressling oberhalb von 98 Gew.-%.

Die Presslinge können aus d-Menthol, 1-Menthol sowie beliebigen Mischungen hiervon hergestellt werden, bevorzugt sind 1-Menthol, d-Menthol und racemisches Menthol.

Das in die Pressung eingesetzte alpha-Menthol kann synthetischen oder natürlichen Ursprungs sein, meist wird synthetisches Material verwendet.

Selbstverständlich können auch beliebige Mischungen von synthetischem und natürlichem Menthol sowie von racemischem und enantiomerenreinem Menthol eingesetzt werden.

Das zur Herstellung der Presslinge verwendete alpha-Menthol kann in beliebiger fester Form vorliegen, bevorzugt sind Kristalle. Diese Kristalle weisen bevorzugt Kristallgrößen im Bereich von 50 - 500 µm, besonders bevorzugt im Bereich von 100 bis 300 µm und ganz besonders bevorzugt im Bereich von 150 bis 250 µm auf.

Der Anteil an kristallisiertem alpha-Menthol liegt bevorzugt bei mindestens 80 Gew.-%, besonders bevorzugt bei mindestens 90 Gew.-% und besonders bevorzugt bei mindestens 95 Gew.-%, bezogen auf den Gesamtgehalt an alpha-Menthol im Pressling. In einer ganz besonders bevorzugten Ausführungsform wird das gesamte alpha-Menthol in Form von kristallisiertem alpha-Menthol eingesetzt.

Die Presslinge können beispielsweise in Form von Kugeln, Würfeln, Quadern, Kissen, Zylindern, Tabletten, Pellets oder Briketts vorliegen, eine bevorzugte Form sind Pellets.

Die durch Pressung (Kompaktierung) gebildeten erfindungsgemäßen Presslinge bleiben bei der Lagerung über mehrere Monate formstabil. Ein Verklumpen, Verbacken oder Zusammenwachsen der Presslinge findet nicht statt. Die bequeme und sichere Handhabbarkeit sowie Schüttfähigkeit, Rieselfähigkeit und Dosierbarkeit der erfindungsgemäßen Presslinge ist daher gegeben.

Die Pressung des Menthols kann auf die unterschiedlichste Art und Weise vorgenommen werden. Bewährte Brikettierungsverfahren sind beispielsweise in Chemie - Anlagen + Verfahren 1985, Heft 4, Seiten 51, 54 und 59 beschrieben.

Zur Kompaktierung wird das Menthol meist in einem Pressvorgang in einen Pressling überfährt. Die Maße des Presslings können stark differieren. Im Falle einer Kugel liegt der Durchmesser im Bereich von 1 mm bis 50 mm; bevorzugt bei 5 bis 20 mm. Im Falle eines Pellets liegen Länge und Breite im Bereich von 3 bis 50 mm, bevorzugt bei 5 bis 15 mm. Die Höhe liegt im Bereich von 0,5 bis 20 mm, bevorzugt bei 2 bis 8 mm.

Bei der Kompaktierung wird im Kompaktorgerät üblicherweise eine Presskraft im Bereich von 5 bis 200 kN (Kilo-Newton) angelegt. Bevorzugt ist eine Presskraft von 10 bis 100 kN, besonders bevorzugt von 20 bis 80 kN.

Bei der Kompak-tierung des Menthols sind auf den Durchmesser bezogen bevorzugt Linienpresskräfte von 1,5 - 4 Newton / mm•mm anzuwenden.

Folgendes Beispiel erläutert die Erfindung.

### Beispiel

Über eine Rüttelrinne werden einem Kompaktorgerät (der Firma Bepex GmbH) bestehend aus zwei Kompaktorwalzen (Umfang 60 cm, Breite 10 cm, Fläche 600 cm²) stündlich 150 bis 200 kg synthetische 1-Menthol Kristalle (Gehalt an alpha-Menthol >98,5 Gew.-%) zugeführt. Etwa 70 Gew.-% dieses Materials wiesen Kristallgrößen im Bereich von 150 - 250 µm auf. Der Kompaktor erzeugt bei Raumtemperatur mit einer Presskraft von 50 kN Presslinge in Form von Pellets (Kissenform) mit den Ausmaßen Länge = Breite = 10 mm und Höhe = 6 mm. Die so hergestellten Presslinge zeigten auch nach dreimonatiger Lagerzeit bei Raumtemperatur kein Verklumpen oder Verbacken.

## Patentansprüche

1. Menthol-Presslinge, **dadurch gekennzeichnet, dass** der Gehalt an alpha-Menthol im Pressling bei größer oder gleich 70 Gew.-% liegt.

2. Presslinge nach Anspruch 1, **dadurch gekennzeichnet, dass** der Gehalt an alpha-Menthol im Pressling bei größer oder gleich 80 Gew.-% liegt.

3. Presslinge nach mindestens einem der Ansprüche 1 oder 2, **dadurch gekennzeichnet, dass** es sich um 1-Menthol handelt.

4. Presslinge nach mindestens einem der Ansprüche 1 bis 3, **dadurch gekennzeichnet, dass** der Anteil an kristallisiertem alpha-Menthol mindestens 80 Gew.-% beträgt, bezogen auf den Gesamtgehalt an alpha-Menthol im Pressling.

5. Presslinge nach mindestens einem der Ansprüche 1 bis 4, **dadurch gekennzeichnet, dass** der Anteil an kristallisiertem alpha-Menthol mindestens 90 Gew.-% beträgt, bezogen auf den Gesamtgehalt an alpha-Menthol im Pressling.

6. Presslinge nach mindestens einem der Ansprüche 1 bis 5, **dadurch gekennzeichnet, dass** das gesamte alpha-Menthol in Form von kristallisiertem alpha-Menthol eingesetzt wird.

7. Presslinge nach mindestens einem der Ansprüche 1 bis 6, **dadurch gekennzeichnet, dass** diese in Form von Kugeln, Würfeln, Quadern, Kissen, Zylindern, Tabletten, Pellets oder Briketts vorliegen.

8. Verfahren zur Herstellung von Menthol-Presslingen, **dadurch gekennzeichnet, dass** das zur Herstellung der Presslinge eingesetzte Menthol einen Gehalt an alpha-Menthol von mindestens 70 Gew.-% aufweist.

9. Verfahren nach Anspruch 8, **dadurch gekennzeichnet, dass** das zur Herstellung der Presslinge eingesetzte Menthol einen Gehalt an alpha-Menthol von mindestens 80 Gew.-% aufweist.

10. Verfahren nach Anspruch 8 oder 9, **dadurch gekennzeichnet, dass** der Anteil an kristallisiertem alpha-Menthol mindestens 80 Gew.-% beträgt, bezogen auf den Gesamtgehalt an alpha-Menthol im Pressling.

11. Verfahren nach mindestens einem der Ansprüche 8 bis 10, **dadurch gekennzeichnet, dass** der Anteil an kristallisiertem alpha-Menthol mindestens 90 Gew.-% beträgt, bezogen auf den Gesamtgehalt an alpha-Menthol im Pressling.

12. Verwendung von Presslingen gemäß mindestens einem der Ansprüche 1 bis 7 zur Einarbeitung in Produkte.

## Claims

1. Menthol mouldings, **characterized in that** the concentration of alpha-menthol in the moulding is greater than or equal to 70 wt. %.

2. Mouldings according to Claim 1, **characterized in that** the concentration of alpha-menthol in the moulding is greater than or equal to 80 wt. %.

3. Mouldings according to at least either of Claims 1 and 2, **characterized in that** the menthol is 1-menthol.

4. Mouldings according to at least one of Claims 1 to 3, **characterized in that** the proportion of crystallized alpha-menthol is at least 80 wt. %, with respect to the entire amount of alpha-menthol in the moulding.

5. Mouldings according to at least one of Claims 1 to 4, **characterized in that** the proportion of crystallized alpha-menthol is at least 90 wt. %, with respect to the entire amount of alpha-menthol in the moulding.

6. Mouldings according to at least one of Claims 1 to 5, **characterized in that** all of the alpha-menthol used is in the form of crystallized alpha-menthol.

7. Mouldings according to at least one of Claims 1 to 6, **characterized in that** these are in the form of spheres, cubes, cuboids, cushions, cylinders, tablets, pellets or briquettes.

8. Process for the production of menthol mouldings, **characterized in that** the menthol used to produce the mouldings has a concentration of alpha-menthol of at least 70 wt. %.

9. Process according to Claim 8, **characterized in that** the menthol used to produce the mouldings has a concentration of alpha-menthol of at least 80 wt. %.

10. Process according to Claim 8 or 9, **characterized in that** the proportion of crystallized alpha-menthol is at least 80 wt. %, with respect to the entire amount of alpha-menthol in the moulding.

11. Process according to at least one of Claims 8 to 10, **characterized in that** the proportion of crystallized alpha-menthol is at least 90 wt. %, with respect to the entire amount of alpha-menthol in the moulding.

12. Use of mouldings according to at least one of Claims 1 to 7 for forming into products.

## Revendications

1. Comprimés au menthol, **caractérisés en ce que** la teneur en alpha-menthol dans le comprimé est supérieure ou égale à 70 % en poids.

2. Comprimés selon la revendication 1, **caractérisés en ce que** la teneur en alpha-menthol dans le comprimé est supérieure ou égale à 80 % en poids.

3. Comprimés selon au moins une des revendications 1 et 2, **caractérisés en ce qu'**il s'agit de 1-menthol.

4. Comprimés selon au moins une des revendications 1 à 3, **caractérisés en ce que** la proportion d'alpha-menthol cristallisé est d'au moins 80 % en poids, par rapport à la teneur totale en alpha-menthol dans le comprimé.

5. Comprimés selon au moins une des revendications 1 à 4, **caractérisés en ce que** la proportion d'alpha-menthol cristallisé est d'au moins 90 % en poids, par rapport à la teneur totale en alpha-menthol dans le comprimé.

6. Comprimés selon au moins une des revendications 1 à 5, **caractérisés en ce que** l'alpha-menthol total est utilisé sous forme d'alpha-menthol cristallisé.

7. Comprimés selon au moins une des revendications 1 à 6, **caractérisés en ce que** ceux-ci se présentent sous la forme de billes, de dés, de parallélépipèdes, de coussins, de cylindres, de pastilles, de pellets ou de briquettes.

8. Procédé de fabrication de comprimés au menthol, **caractérisé en ce que** le menthol utilisé pour la fabrication des comprimés présente une teneur en alpha-menthol d'au moins 70 % en poids.

9. Procédé selon la revendication 8, **caractérisé en ce que** le menthol utilisé pour la préparation des comprimés présente une teneur en alpha-menthol d'au moins 80 % en poids.

10. Procédé selon la revendication 8 ou 9, **caractérisé en ce que** la proportion d'alpha-menthol cristallisé est d'au moins 80 % en poids, par rapport à la teneur totale en alpha-menthol dans le comprimé.

11. Procédé, selon au moins une des revendications 8 à 10, **caractérisé en ce que** la proportion d'alpha-menthol cristallisé est d'au moins 90 % en poids, par rapport à la teneur totale en alpha-menthol dans le comprimé.

12. Utilisation de comprimés selon au moins une des revendications 1 à 7 pour incorporation dans des produits.
